(19) Europäisches Patentamt
European Patent Office
Office européen des brevets

(11) Veröffentlichungsnummer : **0 385 357 B1**

(12) # EUROPÄISCHE PATENTSCHRIFT

(45) Veröffentlichungstag der Patentschrift : **29.04.92 Patentblatt 92/18**

(21) Anmeldenummer : **90103711.9**

(22) Anmeldetag : **26.02.90**

(51) Int. Cl.5 : **C07C 69/734,** C07C 251/48, C07C 323/12, A01N 37/44, A01N 37/38

(54) **Ortho-substituierte 1-Naphthylether und diese enthaltende Fungizide.**

Die Akte enthält technische Angaben, die nach dem Eingang der Anmeldung eingereicht wurden und die nicht in dieser Patentschrift enthalten sind.

(30) Priorität : **28.02.89 DE 3906160**

(43) Veröffentlichungstag der Anmeldung : **05.09.90 Patentblatt 90/36**

(45) Bekanntmachung des Hinweises auf die Patenterteilung : **29.04.92 Patentblatt 92/18**

(84) Benannte Vertragsstaaten : **AT BE CH DE DK ES FR GB GR IT LI NL SE**

(56) Entgegenhaltungen :
**EP-A- 0 178 826**
**EP-A- 0 212 859**
**EP-A- 0 251 082**
**EP-A- 0 253 213**
**EP-A- 0 254 426**
**EP-A- 0 336 211**
**US-A- 3 978 116**

(73) Patentinhaber : **BASF Aktiengesellschaft**
**Carl-Bosch-Strasse 38**
**W-6700 Ludwigshafen (DE)**

(72) Erfinder : **Wenderoth, Bernd, Dr.**
**Schwalbenstrasse 26**
**W-6840 Lampertheim (DE)**
Erfinder : **Sauter, Hubert, Dr.**
**Neckarpromenade 20**
**W-6800 Mannheim 1 (DE)**
Erfinder : **Harreus, Albrecht, Dr.**
**Teichgasse 13**
**W-6700 Ludwigshafen (DE)**
Erfinder : **Roehl, Franz, Dr.**
**Karl-Otto-Braun-Strasse 8**
**W-6700 Ludwigshafen (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**

EP 0 385 357 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue ortho-substituierte 1-Naphthylether, ihre Herstellung, diese enthaltende Fungizide und ihre Verwendung als Fungizide.

Es ist bekannt, Enolether (EP-A-178 826, 212 859) oder Oximether wie zum Beispiel das 2-Benzyloxyphenyl-glyoxylsäuremethylester-O-methyloxim als Fungizide zu verwenden (EP 253213, 254426). Ihre fungizide Wirkung ist jedoch oft nicht ausreichend.

Es wurde nun gefunden, daß ortho-substituierte 1-Naphthylether der allgemeinen Formel I

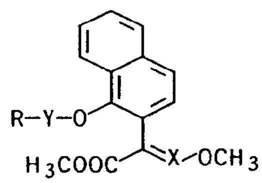

(I)

in der

X N bedeutet,

Y einen gegebenenfalls ungesättigten $C_1$-$C_{12}$-Alkylenrest und

R Aryl, Aryloxy, Arylthio oder Arylalkoxy bedeutet, wobei der aromatische Ring gegebenenfalls durch einen bis fünf der folgenden Reste substituiert ist: Halogen, $C_1$-$C_6$-Alkyl, $C_1$-$C_6$-Alkoxy, $C_1$-$C_4$-Alkylthio, $C_1$-$C_2$-Halogenalkyl, Aryl, Aryl-$C_1$-$C_2$-alkoxy, $C_1$-$C_4$-Alkylcarbonyl, durch $C_1$-$C_4$-Alkyl einfach oder doppelt substituiertes Amino, Cyano oder Nitro,

eine ausgezeichnete fungizide Wirkung haben.

Die in der allgemeinen Formel aufgeführten Reste können beispielsweise folgende Bedeutung haben:

X bedeutet N

Y kann z.B. bedeuten: einen geradkettigen $C_1$-$C_{12}$-Alkylenrest, insbesondere $C_1$-$C_4$-Alkylenrest, (z. B. Methylen, Ethylen, Propylen, Butylen, Pentylen, Hexylen, Heptylen, Octylen, Nonylen, Decylen), einen verzweigten $C_2$-$C_{12}$-Alkylenrest (z.B. Methylethylen), einen $C_4$-$C_{12}$-Alkenylenrest (z.B. Butenylen).

R kann z.B. Aryl (Phenyl), Aryloxy (Phenoxy, 1-Naphthyloxy, 2-Naphthyloxy), Arylthio (Phenylthio) oder Arylalkoxy (Benzyloxy) sein, wobei der aromatische Ring gegebenenfalls durch einen bis fünf (einen bis drei) der folgenden Reste substituiert sein kann:

Halogen (z.B. Fluor, Chlor, Brom, Jod), $C_1$-$C_6$-Alkyl (z.B. Methyl, Ethyl, n- oder iso-Propyl, n-, iso-, sec.- oder tert.-Butyl, n-, iso-, sec.- tert.- oder neo-Pentyl, Hexyl), $C_1$-$C_6$-Alkoxy (z.B. Methoxy, Ethoxy, n- oder iso-Propoxy, n-, iso-, sec.- oder tert.-Butoxy), $C_1$-$C_4$-Alkylthio (z.B. Methylthio, Ethylthio), $C_1$-$C_2$-Halogenalkyl (z.B. Difluormethyl, Trifluormethyl), Aryl (z.B. Phenyl), Aryl-$C_1$-$C_2$-alkoxy (z.B. Benzyloxy), $C_1$-$C_4$-Alkylcarbonyl (z.B. Acetyl), durch $C_1$-$C_4$-Alkyl einfach oder doppelt substituiertes Amino (z.B. Dimethylamino), Cyano, Nitro.

Die neuen Verbindungen der Formel I können aufgrund der C=N-Doppelbinmdung als E/Z-Isomerengemische anfallen, die in üblicher Weise, z.B. durch Kristallisation oder Chromatographie, in die einzelnen Komponenten getrennt werden können. Sowohl die einzelnen Isomeren als auch ihre Gemische werden von der Erfindung umfaßt und sind als Fungizide brauchbar.

Die neuen Verbindungen der allgemeinen Formel I können beispielsweise nach folgenden Verfahren hergestellt werden:

Ausgehend von 1-Naphthol kann durch Friedel-Crafts-Acylierung mit Oxalsäuremethylesterchlorid in einem geeigneten Lösungsmittel (zum Beispiel Dichlormethan) in Gegenwart einer Lewis-Säure (zum Beispiel Titantetrachlorid) der literaturbekannte α-Ketoester III synthetisiert werden (O. Piccolo et al, Tetrahedron 42, 885 - 891 (1986)).

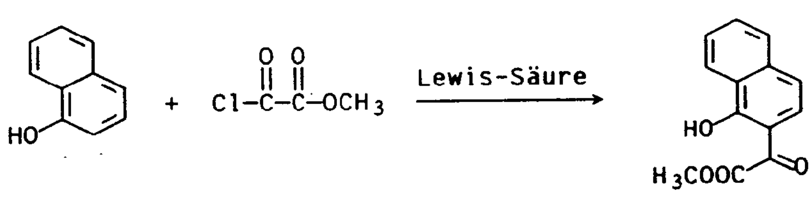


(III)

Ausgehend von der Verbindung der Formel III sind die neuen und wertvollen Zwischenprodukte der Formel IV zugänglich, die von dieser Erfindung ebenfalls umfaßt werden und in der X N bedeutet

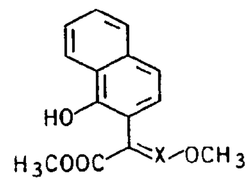

(IV)

Der α-Ketoester III kann in das neue Oximetherderivat der Formel IV (X=N) überführt werden, indem man ihn mit O-Methylhydroxylamin umsetzt, oder indem man zuerst mit Hydroxylamin zum entsprechenden Oxim und danach mit einem Methylierungsmittel wie z.B. Methyljodid oder Dimethylsulfat umsetzt. (vgl. EP 253213).

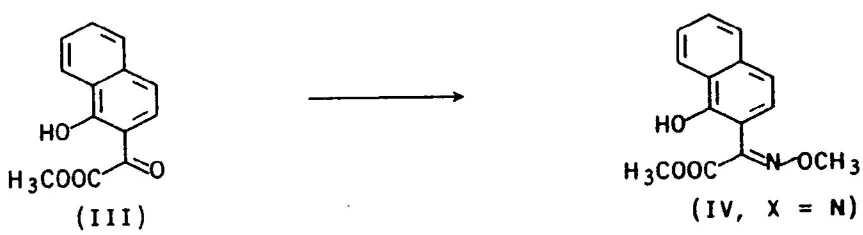

(III) (IV, X = N)

Die neuen Verbindungen der allgemeinen Formel I lassen sich zum Beispiel herstellen, indem man die Verbindung der allgemeinen Formel IV mit Halogeniden der Formel II in einem geeigneten Lösungsmittel wie zum Beispiel Methanol oder N,N-Dimethylformamid nach an sich bekannten Verfahren (vgl. Houben-Weyl, Methoden der organischen Chemie, Band 6/3, S. 54, Thieme-Verlag, Stuttgart 1965) umsetzt.

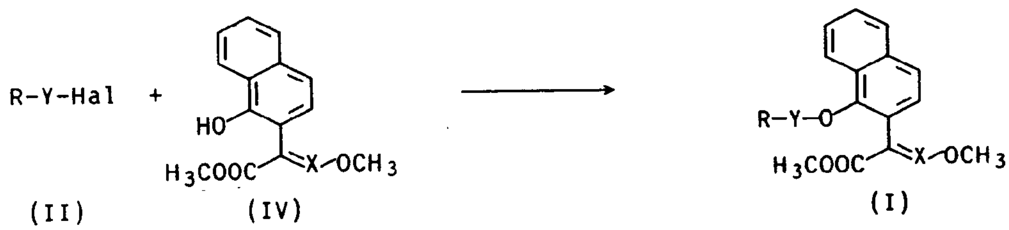

(II) (IV) (I)

R, Y und X haben die obengenannten Bedeutungen.

Hal bedeutet Cl, Br, J.

Die neuen Verbindungen der allgemeinen Formel I lassen sich zum Beispiel auch herstellen, indem man den α-Ketoester der Formel III mit Halogeniden der Formel II in einem geeigneten Lösungsmittel wie zum Beispiel Methanol oder N,N-Dimethylformamid nach an sich bekannten Verfahren (vgl. Houben-Weyl, Methoden der organischen Chemie, Band 6/3, S. 54, Thieme-Verlag, Stuttgart 1965) zu den neuen α-Ketocarbonsäureestern der Formel V umsetzt.

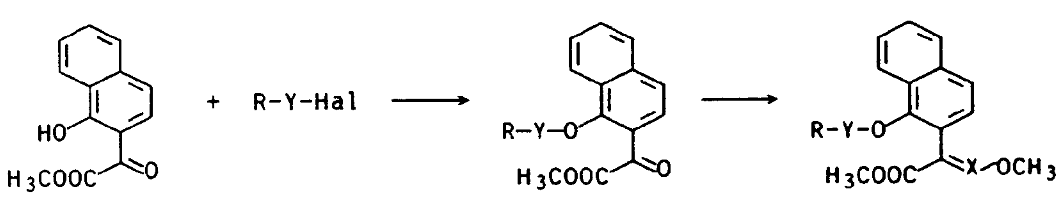

(III) (II) (V) I

Diese Ester der allgemeinen Formel V lassen sich in die erfindungsgemäßen Verbindungen der allgemeinen Formel I überführen; durch Umsetzung mit O-Methylhydroxylamin oder mit Hydroxylamin und anschließender Methylierung mit einem Methylierungsmittel wie z.B. Methyljodid oder Dimethylsulfat erhält man die neuen Oximetherderivate der Formel I (X=N) (vgl. EP 253213).

Die α-Ketocarbonsäureester der Formel V sind neue und wertvolle Zwischenprodukte und werden von dieser Erfindung ebenfalls erfaßt.

Die Herstellung der neuen Verbindungen der Formeln I und IV wird durch folgende Beispiele erläutert:

## Herstellungsbeispiele

a) Bei -10°C werden unter Stickstoff 28,5 g (0,15 mol) Titantetrachlorid zu 150 ml Methylenchlorid gegeben. Dann werden bei -40°C zuerst 22,3 g (0,15 mol) 1-Naphthol, gelöst in 100 ml Methylenchlorid und anschließend 18,5 g (0,15 mol) Oxalsäuremethylesterchlorid zugetropft. Man rührt noch 30 min bei dieser Temperatur, gibt dann bei Raumtemperatur (20°C) etwas verdünnte Salzsäure zu und gießt auf Eis. Die organische Phase wird 1 x mit $H_2O$ gewaschen, über Natriumsulfat getrocknet und eingeengt. Man erhält 30 g (0,13 mol) 1-Hydroxy-2-naphthylglyoxylsäuremethylester (87 % Ausbeute).

$^1$H-NMR ($CDCl_3$): δ = 4.05(s,3H), 7.25(d,1H), 7.40-7.80(m,4H), 8.45(d,1H), 13.00(s,1H).

b) 69 g (0,26 mol) 1-Hydroxy-2-naphthylglyoxylsäuremethylester und 44 g (0,53 mol) O-Methylhydroxylaminhydrochlorid werden in 800 ml Methanol vorgelegt und 6 h unter Rühren auf Rückflußtemperatur erhitzt. Dann wird eingeengt; der Rückstand wird in Essigester aufgenommen, mit $H_2O$ gewaschen, über Natriumsulfat getrocknet und erneut eingeengt. Man erhält 52 g (0,20 mol) 1-Hydroxy-2-naphthylglyoxylsäuremethylester-O-methyloxim (77 % Ausbeute).

$^1$H-NMR ($CDCl_3$): δ = 4.00(s,3H), 4.05(s,3H), 7.00(d,1H) 7.30(d,1H), 7.40-7.90(m,4H), 8.35(m,1H), 10,90(s,1H).

c) 5 g (19 mmol) 1-Hydroxy-2-naphthylglyoxylsäuremethylester-O-methyloxim, 2,7 g (19 mmol) 2-Fluorbenzylchlorid und 2,6 g (19 mmol) Kaliumcarbonat werden in 65 ml Methanol vorgelegt und 8 h unter Rühren auf Rückflußtemperatur erhitzt. Nach dem Abkühlen auf Raumtemperatur wird vom Feststoff abfiltriert. Das Filtrat wird eingeengt und mit verdünnter Natronlauge versetzt. Dann wird mehrmals mit Ether ausgeschüttelt; die vereinigten Etherphasen werden mit $H_2O$ gewaschen, über Natriumsulfat getrocknet und eingeengt. Man erhält 5 g (13,6 mmol) 1-(2-Fluorbenzyloxy)-2-naphthylglyoxylsäuremethylester-O-methyloxim (Ausbeute 71 %) (Verbindung Nr. 2, Tabelle 1).

$^1$H-NMR ($CDCl_3$): δ = 3.65(s,3H), 4.10(s,3H), 5.10(s,2H), 7.00-8.20(m,10H).

In entsprechender Weise werden die folgenden Verbindungen hergestellt.

Tabelle 1

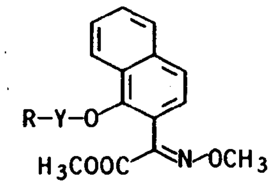

(I, X = N)

| Verb.-Nr. | R | Y | Fp($^oC$) | IR($cm^{-1}$) |
|---|---|---|---|---|
| 1 | $C_6H_5$ | $-CH_2-$ | Harz | 2930, 1743, 1358, 1227, 1061, 1036, 752 |
| 2 | $2-F-C_6H_4$ | $-CH_2-$ | Öl | 2940, 1743, 1493, 1231, 1037, 757 |
| 3 | $3-F-C_6H_4$ | $-CH_2-$ | Harz | 2950, 1743, 1358, 1263, 1037, 752 |
| 4 | $4-F-C_6H_4$ | $-CH_2-$ | Harz | 2940, 1742, 1511, 1225, 1037, 821 |
| 5 | $2,4-F_2-C_6H_3$ | $-CH_2-$ | Harz | 2940, 1742, 1506, 1227, 1101, 1037, 852 |
| 6 | $2-Cl-C_6H_4$ | $-CH_2-$ | Harz | 2940, 1743, 1358, 1226, 1037, 753 |
| 7 | $3-Cl-C_6H_4$ | $-CH_2-$ | Harz | 2940, 1742, 1356, 1226, 1037, 752 |
| 8 | $4-Cl-C_6H_4$ | $-CH_2-$ | Harz | 2940, 1743, 1357, 1227, 1037, 810 |
| 9 | $2,4-Cl_2-C_6H_3$ | $-CH_2-$ | 95 | 2940, 1733, 1474, 1231, 1098, 1037, 815 |
| 10 | $2,6-Cl_2-C_6H_3$ | $-CH_2-$ | | |
| 11 | $3,5-Cl_2-C_6H_3$ | $-CH_2-$ | | |
| 12 | $2,4,6-Cl_3-C_6H_2$ | $-CH_2-$ | | |
| 13 | $2-Br-C_6H_4$ | $-CH_2-$ | Harz | 2940, 1743, 1356, 1226, 1037, 752 |
| 14 | $3-Br-C_6H_4$ | $-CH_2-$ | Harz | 2940, 1742, 1355, 1226, 1061, 1036 |

Tabelle 1 (Fortsetzung)

| Verb.-Nr. | R | Y | Fp(°C) | IR($cm^{-1}$) |
|---|---|---|---|---|
| 15 | $4-Br-C_6H_4$ | $-CH_2-$ | Harz | 2940, 1742, 1356, 1227, 1037, 808 |
| 16 | $2,4-Br_2-C_6H_3$ | $-CH_2-$ | | |
| 17 | $2-J-C_6H_4$ | $-CH_2-$ | Harz | 2945, 1743, 1335, 1225, 1100, 1062, 1037, 750 |
| 18 | $2-CH_3-C_6H_4$ | $-CH_2-$ | Harz | 2930, 1742, 1359, 1226, 1036, 748 |
| 19 | $3-CH_3-C_6H_4$ | $-CH_2-$ | Harz | 2940, 1743, 1355, 1226, 1037, 752 |
| 20 | $4-CH_3-C_6H_4$ | $-CH_2-$ | Harz | 2930, 1743, 1358, 1225, 1060, 1037 |
| 21 | $2,4-(CH_3)_2-C_6H_3$ | $-CH_2-$ | | |
| 22 | $2,5-(CH_3)_2-C_6H_3$ | $-CH_2-$ | Harz | 2920, 1742, 1358, 1225, 1100, 1061, 1036, 818 |
| 23 | $2,4,6-(CH_3)_3-C_6H_2$ | $-CH_2-$ | | |
| 24 | $4-t-C_4H_9-C_6H_4$ | $-CH_2-$ | Harz | 2962, 1745, 1356, 1226, 1100, 1061, 1037, 817 |
| 25 | $4-i-C_3H_7-C_6H_4$ | $-CH_2-$ | | |
| 26 | $4-C_2H_5-C_6H_4$ | $-CH_2-$ | Harz | 2950, 1743, 1358, 1225, 1061, 1037, 819, 752 |
| 27 | $2-CH_3O-C_6H_4$ | $-CH_2-$ | | |
| 28 | $3-CH_3O-C_6H_4$ | $-CH_2-$ | Harz | 2930, 1742, 1357, 1265, 1227, 1037 |
| 29 | $4-CH_3O-C_6H_4$ | $-CH_2-$ | | |
| 30 | $4-C_2H_5O-C_6H_4$ | $-CH_2-$ | | |
| 31 | $4-i-C_3H_7O-C_6H_4$ | $-CH_2-$ | | |
| 32 | $2-CF_3-C_6H_4$ | $-CH_2-$ | | |
| 33 | $3-CF_3-C_6H_4$ | $-CH_2-$ | Harz | 1940, 1743, 1331, 1166, 1126, 1037 |
| 34 | $4-CF_3-C_6H_4$ | $-CH_2-$ | | |
| 35 | $2-CN-C_6H_4$ | $-CH_2-$ | 81-85 | 2940, 2230, 1742, 1360, 1228, 1036, 764 |
| 36 | $3-CN-C_6H_4$ | $-CH_2-$ | Harz | 2940, 2225, 1741, 1358, 1229, 1036, 796 |
| 37 | $4-CN-C_6H_4$ | $-CH_2-$ | Harz | 2930, 2220, 1741, 1359, 1228, 1036, 819 |
| 38 | $2-NO_2-C_6H_4$ | $-CH_2-$ | | |

Tabelle 1 (Fortsetzung)

| Verb.-Nr. | R | Y | Fp($^oC$) | IR($cm^{-1}$) |
|---|---|---|---|---|
| 39 | $3-NO_2-C_6H_4$ | $-CH_2-$ | Harz | 2940, 1737, 1531, 1350, 1229, 1038, 730 |
| 40 | $4-NO_2-C_6H_4$ | $-CH_2-$ | | |
| 41 | $3-C_6H_5-C_6H_4$ | $-CH_2-$ | | |
| 42 | $4-C_6H_5-C_6H_4$ | $-CH_2-$ | | |
| 43 | $4-C_6H_5O-C_6H_4$ | $-CH_2-$ | | |
| 44 | $2-F, 3-CH_3-C_6H_3$ | $-CH_2-$ | | |
| 45 | $2-Cl, 3-CH_3-C_6H_3$ | $-CH_2-$ | Harz | 2940, 1743, 1354, 1225, 1102, 1063, 1037, 772 |
| 46 | $2-Br, 3-CH_3-C_6H_3$ | $-CH_2-$ | 109 | 2935, 1738, 1353, 1225, 1102, 1062, 1032, 772 |
| 47 | $2-Cl, 3-i-C_3H_7-C_6H_3$ | $-CH_2-$ | Harz | 2964, 1744, 1355, 1225, 1102, 1063, 1037, 756 |
| 48 | $2-CH_3, 3-i-C_3H_7-C_6H_3$ | $-CH_2-$ | | |
| 49 | $2-CH_3, 3-C_6H_5-C_6H_3$ | $-CH_2-$ | | |
| 50 | $2-Cl, 3-C_6H_5-C_6H_3$ | $-CH_2-$ | | |
| 51 | $2-CH_3O, 3-i-C_3H_7-C_6H_3$ | $-CH_2-$ | | |
| 52 | $2,4,5-Cl_3-C_6H_2$ | $-CH_2-$ | | |
| 53 | $2,4,5-(CH_3)_3-C_6H_2$ | $-CH_2-$ | | |
| 54 | $2-CH_3O, 5-NO_2-C_6H_3$ | $-CH_2-$ | | |
| 55 | $2,5-Cl_2-C_6H_3$ | $-CH_2-$ | | |
| 56 | $2,5-F_2-C_6H_3$ | $-CH_2-$ | | |
| 57 | $2-Cl, 5-CH_3-C_6H_3$ | $-CH_2-$ | | |
| 58 | $2-F, 3-CH_3-C_6H_3$ | $-CH_2-$ | | |
| 59 | $2,3-F_2-C_6H_3$ | $-CH_2-$ | | |
| 60 | $2,3-Cl_2-C_6H_3$ | $-CH_2-$ | | |
| 61 | $2-F, 3-Cl-C_6H_3$ | $-CH_2-$ | | |
| 62 | $2-F, 4-Br-C_6H_3$ | $-CH_2-$ | | |

Tabelle 1 (Fortsetzung)

| Verb.-Nr. | R | Y | Fp(°C) | IR($cm^{-1}$) |
|---|---|---|---|---|
| 63 | $2,6-F_2-C_6H_3$ | $-CH_2-$ | | |
| 64 | $2-F,6-Cl-C_6H_3$ | $-CH_2-$ | | |
| 65 | $2-Cl,6-CF_3-C_6H_3$ | $-CH_2-$ | | |
| 66 | $2,3,4,5,6-(CH_3)_5-C_6$ | $-CH_2-$ | | |
| 67 | $2,3,4,5,6-F_5-C_6$ | $-CH_2-$ | | |
| 68 | $2-CH_3,4-Cl-C_6H_3$ | $-CH_2-$ | | |
| 69 | $2-Cl,4-CH_3-C_6H_3$ | $-CH_2-$ | | |
| 70 | $2-CH_3O,4-CH_3-C_6H_3$ | $-CH_2-$ | | |
| 71 | $4-CH_3O,2-CH_3-C_6H_3$ | $-CH_2-$ | | |
| 72 | $C_6H_5-O-$ | $-CH_2-CH_2-$ | | |
| 73 | $2-F-C_6H_4-O-$ | $-CH_2-CH_2-$ | | |
| 74 | $3-F-C_6H_4-O-$ | $-CH_2-CH_2-$ | | |
| 75 | $4-F-C_6H_4-O-$ | $-CH_2-CH_2-$ | | |
| 76 | $2-Cl-C_6H_4-O-$ | $-CH_2-CH_2-$ | | |
| 77 | $3-Cl-C_6H_4-O-$ | $-CH_2-CH_2-$ | | |
| 78 | $4-Cl-C_6H_4-O-$ | $-CH_2-CH_2-$ | | |
| 79 | $2-CH_3-C_6H_4-O-$ | $-CH_2-CH_2-$ | | |
| 80 | $3-CH_3-C_6H_4-O-$ | $-CH_2-CH_2-$ | | |
| 81 | $4-CH_3-C_6H_4-O-$ | $-CH_2-CH_2-$ | | |
| 82 | $2-CH_3O-C_6H_4-O-$ | $-CH_2-CH_2-$ | | |
| 83 | $3-CH_3O-C_6H_4-O-$ | $-CH_2-CH_2-$ | | |
| 84 | $4-CH_3O-C_6H_4-O-$ | $-CH_2-CH_2-$ | | |
| 85 | $4-t-C_4H_9-C_6H_4-O-$ | $-CH_2-CH_2-$ | | |
| 86 | $4-C_2H_5O-C_6H_4-O-$ | $-CH_2-CH_2-$ | | |

Tabelle 1 (Fortsetzung)

| Verb.-Nr. | R | Y | Fp($^oC$) | IR($cm^{-1}$) |
|---|---|---|---|---|
| 87 | $4-CF_3-C_6H_4-O-$ | $-CH_2-CH_2-$ | | |
| 88 | $4-C_6H_5-C_6H_4-O-$ | $-CH_2-CH_2-$ | | |
| 89 | $4-(C_6H_5-CH_2-O-)-C_6H_4-O-$ | $-CH_2-CH_2$ | | |
| 90 | $2,4,6-Cl_3-C_6H_2-O-$ | $-CH_2-CH_2-$ | | |
| 91 | $2,4,6-(CH_3)_3-C_6H_2-O-$ | $-CH_2-CH_2-$ | | |
| 92 | 1-Naphthyl-O- | $-CH_2-CH_2-$ | | |
| 93 | 2-Naphthyl-O- | $-CH_2-CH_2-$ | | |
| 94 | $2-Br-C_6H_4-O-$ | $-CH_2-CH_2-$ | | |
| 95 | $3-Br-C_6H_4-O-$ | $-CH_2-CH_2-$ | | |
| 96 | $4-Br-C_6H_4-O-$ | $-CH_2-CH_2-$ | | |
| 97 | $2-CF_3-C_6H_4-O-$ | $-CH_2-CH_2-$ | | |
| 98 | $3-CF_3-C_6H_4-O-$ | $-CH_2-CH_2-$ | | |
| 99 | $4-CN-C_6H_4-O-$ | $-CH_2-CH_2-$ | | |
| 100 | $4-CH_3CO-C_6H_4-O-$ | $-CH_2-CH_2-$ | | |
| 101 | $3-(CH_3)_2N-C_6H_4-O-$ | $-CH_2-CH_2-$ | | |
| 102 | $4-CH_3S-C_6H_4-O-$ | $-CH_2-CH_2-$ | | |
| 103 | $2-C_2H_5O-C_6H_4-O-$ | $-CH_2-CH_2-$ | | |
| 104 | $4-t-C_4H_9-C_6H_4-O-$ | $-CH_2-CH_2-$ | | |
| 105 | $2,4-Cl_2-H_6H_3-O-$ | $-CH_2-CH_2-$ | | |
| 106 | $3,4-Cl_2-C_6H_3-O-$ | $-CH_2-CH_2-$ | | |
| 107 | $2,5-Cl_2-C_6H_3-O-$ | $-CH_2-CH_2-$ | | |
| 108 | $2,6-Cl_2-C_6H_3-O-$ | $-CH_2-CH_2-$ | | |
| 109 | $2,4,5-Cl_3-C_6H_2-O-$ | $-CH_2-CH_2-$ | | |
| 110 | $2-Cl,4-F-C_6H_3-O-$ | $-CH_2-CH_2-$ | | |

Tabelle 1 (Fortsetzung)

| Verb.-Nr. | R | Y | Fp($^oC$) | IR($cm^{-1}$) |
|---|---|---|---|---|
| 111 | $2-Cl,4-Br-C_6H_3-O-$ | $-CH_2-CH_2-$ | | |
| 112 | $2,4-(CH_3)_2-C_6H_3-O-$ | $-CH_2-CH_2-$ | | |
| 113 | $2-CH_3,4-t-C_4H_9-C_6H_3-O$ | $-CH_2-CH_2-$ | | |
| 114 | $2-CH_3,4-Cl-C_6H_3-O-$ | $-CH_2-CH_2-$ | | |
| 115 | $2-CH_3,4,6-Cl_2-C_6H_2-O-$ | $-CH_2-CH_2-$ | | |
| 116 | $2,6-(CH_3)_2-C_6H_3-O-$ | $-CH_2-CH_2-$ | | |
| 117 | $2,5,6-(CH_3)_3-C_6H_2-O-$ | $-CH_2-CH_2-$ | | |
| 118 | $2,4,5-(CH_3)_3-C_6H_2-O-$ | $-CH_2-CH_2-$ | | |
| 119 | $2-i-C_3H_7,5-CH_3-C_6H_3-O-$ | $-CH_2-CH_2-$ | | |
| 120 | $3,5-(C_2H_5)_2-C_6H_3-O-$ | $-CH_2-CH_2-$ | | |
| 121 | $2,4,6-(sec-C_4H_9)_3-C_6H_2-O-$ | $-CH_2-CH_2-$ | | |
| 122 | $3,5-(CH_3O)_2-C_6H_3-O-$ | $-CH_2-CH_2-$ | | |
| 123 | $2-CH_3O,4-CH_3-C_6H_3-O-$ | $-CH_2-CH_2-$ | | |
| 124 | $2,6-(CH_3)_2,4-CH_3S-C_6H_2-O-$ | $-CH_2-CH_2-$ | | |
| 125 | $2-Cl,5-CH_3O-C_6H_3-O-$ | $-CH_2-CH_2-$ | | |
| 126 | $2-CH_3,4-CH_3S-C_6H_3-O-$ | $-CH_2-CH_2-$ | | |
| 127 | $2,6-(CH_3O)_2-C_6H_3-O-$ | $-CH_2-CH_2-$ | | |
| 128 | $2-Cl,4-C_6H_5-C_6H_3-O-$ | $-CH_2-CH_2-$ | | |
| 129 | $2-Cl,4-CH_3-C_6H_3-O-$ | $-CH_2-CH_2-$ | | |
| 130 | $2-CH_3,6-i-C_3H_7-C_6H_3-O-$ | $-CH_2-CH_2-$ | | |
| 131 | $3-t-C_4H_9,4-CH_3O-C_6H_3-O-$ | $-CH_2-CH_2-$ | | |
| 132 | $2,4-(CH_3)_2$-1-Naphthyl-O- | $-CH_2-CH_2-$ | | |
| 133 | $C_6H_5-S$ | $-CH_2-CH_2-$ | | |
| 134 | $4-Cl-C_6H_4-S$ | $-CH_2-CH_2-$ | | |

Tabelle 1 (Fortsetzung)

| Verb.-Nr. | R | Y | Fp(°C) | IR($cm^{-1}$) |
|---|---|---|---|---|
| 135 | $2,4,6-Cl_3-C_6H_2-O-$ | $-CH_2-CH(CH_3)-$ | | |
| 136 | $C_6H_5-O-$ | $-CH_2-CH(CH_3)-$ | | |
| 137 | $C_6H_5-O-$ | $-(CH_2)_3-$ | | |
| 138 | $2-F-C_6H_4-O-$ | $-(CH_2)_3-$ | | |
| 139 | $3-F-C_6H_4-O-$ | $-(CH_2)_3-$ | | |
| 140 | $4-F-C_6H_4-O-$ | $-(CH_2)_3-$ | | |
| 141 | $2-Cl-C_6H_4-O-$ | $-(CH_2)_3-$ | | |
| 142 | $3-Cl-C_6H_4-O-$ | $-(CH_2)_3-$ | | |
| 143 | $4-Cl-C_6H_4-O-$ | $-(CH_2)_3-$ | | |
| 144 | $2-CH_3-C_6H_4-O-$ | $-(CH_2)_3-$ | | |
| 145 | $3-CH_3-C_6H_4-O-$ | $-(CH_2)_3-$ | | |
| 146 | $4-CH_3-C_6H_4-O-$ | $-(CH_2)_3-$ | | |
| 147 | $2-CH_3O-C_6H_4-O-$ | $-(CH_2)_3-$ | | |
| 148 | $3-CH_3O-C_6H_4-O-$ | $-(CH_2)_3-$ | | |
| 149 | $4-CH_3O-C_6H_4-O-$ | $-(CH_2)_3-$ | | |
| 150 | $4-t-C_4H_9-C_6H_4-O-$ | $-(CH_2)_3-$ | | |
| 151 | $4-C_2H_5O-C_6H_4-O-$ | $-(CH_2)_3-$ | | |
| 152 | $4-CF_3-C_6H_4-O-$ | $-(CH_2)_3-$ | | |
| 153 | $4-C_6H_5-C_6H_4-O-$ | $-(CH_2)_3-$ | | |
| 154 | $4-(C_6H_5-CH_2-O)-C_6H_4-O-$ | $-(CH_2)_3-$ | | |
| 155 | $2,4,6-Cl_3-C_6H_2-O-$ | $-(CH_2)_3-$ | | |
| 156 | $2,4,6-(CH_3)_3-C_6H_2-O-$ | $-(CH_2)_3-$ | | |
| 157 | 1-Naphthyl-O- | $-(CH_2)_3-$ | | |
| 158 | 2-Naphthyl-O- | $-(CH_2)_3-$ | | |

Tabelle 1 (Fortsetzung)

| Verb.-Nr. | R | Y | Fp(°C) | IR($cm^{-1}$) |
|---|---|---|---|---|
| 159 | 2,4-$Cl_2$-$C_6H_3$-O- | -$(CH_2)_3$- | | |
| 160 | 3,5-$Cl_2$-$C_6H_3$-O- | -$(CH_2)_3$- | | |
| 161 | 3,4-$Cl_2$-$C_6H_3$-O- | -$(CH_2)_3$- | | |
| 162 | 2,6-$(CH_3)_2$-$C_6H_3$-O- | -$(CH_2)_3$- | | |
| 163 | 2,4-$(CH_3)_2$-$C_6H_3$-O- | -$(CH_3)_3$- | | |
| 164 | 3-$CF_3$-$C_6H_4$-O- | -$(CH_2)_3$- | | |
| 165 | 2-Cl,4-$CH_3$-$C_6H_3$-O- | -$(CH_2)_3$- | | |
| 166 | $C_6H_5$-S | -$(CH_2)_3$- | | |
| 167 | $C_6H_5$-$CH_2$-O- | -$(CH_2)_3$- | | |
| 168 | $C_6H_5$-O- | -$(CH_2)_4$- | | |
| 169 | 2-F-$C_6H_4$-O- | -$(CH_2)_4$- | | |
| 170 | 3-F-$C_6H_4$-O- | -$(CH_2)_4$- | | |
| 171 | 4-F-$C_6H_4$-O- | -$(CH_2)_4$- | | |
| 172 | 2-$CH_3$-$C_6H_4$-O- | -$(CH_2)_4$- | | |
| 173 | 3-$CH_3$-$C_6H_4$-O- | -$(CH_2)_4$- | | |
| 174 | 4-$CH_3$-$C_6H_4$-O- | -$(CH_2)_4$- | | |
| 175 | 2-$CH_3O$-$C_6H_4$-O- | -$(CH_2)_4$- | | |
| 176 | 3-$CH_3O$-$C_6H_4$-O- | -$(CH_2)_4$- | | |
| 177 | 4-$CH_3O$-$C_6H_4$-O- | -$(CH_2)_4$- | | |
| 178 | 4-t-$C_4H_9$-$C_6H_4$-O- | -$(CH_2)_4$- | | |
| 179 | 4-$C_2H_5O$-$C_6H_4$-O- | -$(CH_2)_4$- | | |
| 180 | 4-$CF_3$-$C_6H_4$-O- | -$(CH_2)_4$- | | |
| 181 | 4-$C_6H_5$-$C_6H_4$-O- | -$(CH_2)_4$- | | |
| 182 | 4-($C_6H_5$-$CH_2$-O)-$C_6H_4$- | -$(CH_2)_4$- | | |

Tabelle 1 (Fortsetzung)

| Verb.-Nr. | R | Y | Fp($^oC$) | IR($cm^{-1}$) |
|---|---|---|---|---|
| 183 | $2,4,6-Cl_3-C_6H_2-O-$ | $-(CH_2)_4-$ | | |
| 184 | $2,4,6-(CH_3)_3-C_6H_2-O-$ | $-(CH_2)_4-$ | | |
| 185 | 1-Naphthyl-O- | $-(CH_2)_4-$ | | |
| 186 | 2-Napthyl-O- | $-(CH_2)_4-$ | | |
| 187 | $3-CF_3-C_6H_4-O-$ | $-(CH_2)_4-$ | | |
| 188 | $3-iso-C_3H_7-C_6H_4-O-$ | $-(CH_2)_4-$ | | |
| 189 | $4-iso-C_3H_7-C_6H_4-O-$ | $-(CH_2)_4-$ | | |
| 190 | $3-t-C_4H_9-C_6H_4-O-$ | $-(CH_2)_4-$ | | |
| 191 | $4-t-C_4H_9-C_6H_4-O-$ | $-(CH_2)_4-$ | | |
| 192 | $3-C_6H_5-C_6H_4-O-$ | $-(CH_2)_4-$ | | |
| 193 | $2,4-Cl_2-C_6H_3-O-$ | $-(CH_2)_4-$ | | |
| 194 | $3,4-Cl_2-C_6H_3-O-$ | $-(CH_2)_4-$ | | |
| 195 | $3,5-Cl_2-C_6H_3-O-$ | $-(CH_2)_4-$ | | |
| 196 | $2,6-Cl_2-C_6H_3-O-$ | $-(CH_2)_4-$ | | |
| 197 | $2-Cl,4-CH_3-C_6H_3-O-$ | $-(CH_2)_4-$ | | |
| 198 | $2,6-(CH_3)_2-C_6H_3-O-$ | $-(CH_2)_4-$ | | |
| 199 | $2,5-(CH_3)_2-C_6H_3-O-$ | $-(CH_2)_4-$ | | |
| 200 | $4-CN-C_6H_4-O-$ | $-(CH_2)_4-$ | | |
| 201 | $C_6H_5-S-$ | $-(CH_2)_4-$ | | |
| 202 | $C_6H_5-CH_2-O-$ | $-(CH_2)_4-$ | | |
| 203 | $C_6H_5-O-$ | $-CH_2-CH=CH-CH_2-$ | | |
| 204 | $2-F-C_6H_4-O-$ | $-CH_2-CH=CH-CH_2-$ | | |
| 205 | $3-F-C_6H_4-O-$ | $-CH_2-CH=CH-CH_2-$ | | |
| 206 | $4-F-C_6H_4-O-$ | $-CH_2-CH=CH-CH_2-$ | | |

Tabelle 1 (Fortsetzung)

| Verb.-Nr. | R | Y | Fp(°C) | IR($cm^{-1}$) |
|---|---|---|---|---|
| 207 | 2-Cl-$C_6H_4$-O- | $-CH_2-CH=CH-CH_2-$ | | |
| 208 | 3-Cl-$C_6H_4$-O- | $-CH_2-CH=CH-CH_2-$ | | |
| 209 | 4-Cl-$C_6H_4$-O- | $-CH_2-CH=CH-CH_2-$ | | |
| 210 | $C_6H_5$-O- | $-(CH_2)_5-$ | | |
| 211 | 2-Cl-$C_6H_4$-O- | $-(CH_2)_5-$ | | |
| 212 | 3-Cl-$C_6H_4$-O- | $-(CH_2)_5-$ | | |
| 213 | 4-Cl-$C_6H_4$-O- | $-(CH_2)_5-$ | | |
| 214 | 2-F-$C_6H_4$-O- | $-(CH_2)_5-$ | | |
| 215 | 3-F-$C_6H_4$-O- | $-(CH_2)_5-$ | | |
| 216 | 4-F-$C_6H_4$-O- | $-(CH_2)_5-$ | | |
| 217 | 2-$CH_3$-$C_6H_4$-O- | $-(CH_2)_5-$ | | |
| 218 | 3-$CH_3$-$C_6H_4$-O- | $-(CH_2)_5-$ | | |
| 219 | 4-$CH_3$-$C_6H_4$-O- | $-(CH_2)_5-$ | | |
| 220 | 2-$CH_3O$-$C_6H_4$-O | $-(CH_2)_5-$ | | |
| 221 | 3-$CH_3O$-$C_6H_4$-O | $-(CH_2)_5-$ | | |
| 222 | 4-$CH_3O$-$C_6H_4$-O | $-(CH_2)_5-$ | | |
| 223 | 4-t-$C_4H_9$-$C_6H_4$-O- | $-(CH_2)_5-$ | | |
| 224 | 4-$C_2H_5O$-$C_6H_4$-O- | $-(CH_2)_5-$ | | |
| 225 | 4-$CF_3$-$C_6H_4$-O- | $-(CH_2)_5-$ | | |
| 226 | 4-$C_6H_5$-$C_6H_4$-O- | $-(CH_2)_5-$ | | |
| 227 | 4-($C_6H_5$-$CH_2$-O)-$C_6H_4$-O- | $-(CH_2)_5-$ | | |
| 228 | 2,4,6-$Cl_3$-$C_6H_2$-O- | $-(CH_2)_5-$ | | |
| 229 | 2,4,6-$(CH_3)_3$-$C_6H_2$-O- | $-(CH_2)_5-$ | | |
| 230 | 1-Naphthyl-O- | $-(CH_2)_5-$ | | |

Tabelle 1 (Fortsetzung)

| Verb.-Nr. | R | Y | Fp($^oC$) | IR($cm^{-1}$) |
|---|---|---|---|---|
| 231 | 2-Naphthyl-O- | $-(CH_2)_5-$ | | |
| 232 | $C_6H_5-CH_2-O-$ | $-(CH_2)_5-$ | | |
| 233 | $C_6H_5-O-$ | $-(CH_2)_6-$ | | |
| 234 | $2-Cl-C_6H_4-O-$ | $-(CH_2)_6-$ | | |
| 235 | $3-Cl-C_6H_4-O-$ | $-(CH_2)_6-$ | | |
| 236 | $4-Cl-C_6H_4-O-$ | $-(CH_2)_6-$ | | |
| 237 | $2-F-C_6H_4-O-$ | $-(CH_2)_6-$ | | |
| 238 | $3-F-C_6H_4-O-$ | $-(CH_2)_6-$ | | |
| 239 | $4-F-C_6H_4-O-$ | $-(CH_2)_6-$ | | |
| 240 | $2-CH_3-C_6H_4-O-$ | $-(CH_2)_6-$ | | |
| 241 | $3-CH_3-C_6H_4-O-$ | $-(CH_2)_6-$ | | |
| 242 | $4-CH_3-C_6H_4-O-$ | $-(CH_2)_6-$ | | |
| 243 | $2-CH_3O-C_6H_4-O-$ | $-(CH_2)_6-$ | | |
| 244 | $3-CH_3O-C_6H_4-O-$ | $-(CH_2)_6-$ | | |
| 245 | $4-CH_3O-C_6H_4-O-$ | $-(CH_2)_6-$ | | |
| 246 | $4-t-C_4H_9-C_6H_4-O-$ | $-(CH_2)_6-$ | | |
| 247 | $4-C_2H_5O-C_6H_4-O$ | $-(CH_2)_6-$ | | |
| 248 | $4-CF_3-C_6H_4-O-$ | $-(CH_2)_6-$ | | |
| 249 | $4-C_6H_5-C_6H_4-O-$ | $-(CH_2)_6-$ | | |
| 250 | $4-(C_6H_5-CH_2-O)-C_6H_4-O-$ | $-(CH_2)_6-$ | | |
| 251 | $2,4,6-Cl_3-C_6H_2-O-$ | $-(CH_2)_6-$ | | |
| 252 | $2,4,6-(CH_3)_3-C_6H_2-O-$ | $-(CH_2)_6-$ | | |
| 253 | 1-Naphthyl-O- | $-(CH_2)_6-$ | | |
| 254 | 2-Naphthyl-O- | $-(CH_2)_6-$ | | |

Tabelle 1 (Fortsetzung)

| Verb.-Nr. | R | Y | Fp($^oC$) | IR($cm^{-1}$) |
|---|---|---|---|---|
| 255 | $C_6H_5-CH_2-O-$ | $-(CH_2)_6-$ | | |
| 256 | $C_6H_5-O-$ | $-(CH_2)_7-$ | | |
| 257 | $2-Cl-C_6H_4-O-$ | $-(CH_2)_7-$ | | |
| 258 | $3-Cl-C_6H_4-O-$ | $-(CH_2)_7-$ | | |
| 259 | $4-Cl-C_6H_4-O-$ | $-(CH_2)_7-$ | | |
| 260 | $2-F-C_6H_4-O-$ | $-(CH_2)_7-$ | | |
| 261 | $3-F-C_6H_4-O-$ | $-(CH_2)_7-$ | | |
| 262 | $4-F-C_6H_4-O-$ | $-(CH_2)_7-$ | | |
| 263 | $2-CH_3-C_6H_4-O-$ | $-(CH_2)_7-$ | | |
| 264 | $3-CH_3-C_6H_4-O-$ | $-(CH_2)_7-$ | | |
| 265 | $4-CH_3-C_6H_4-O-$ | $-(CH_2)_7-$ | | |
| 266 | $2-CH_3O-C_6H_4-O-$ | $-(CH_2)_7-$ | | |
| 267 | $3-CH_3O-C_6H_4-O-$ | $-(CH_2)_7-$ | | |
| 268 | $4-CH_3O-C_6H_4-O-$ | $-(CH_2)_7-$ | | |
| 269 | $4-t-C_4H_9-C_6H_4-O-$ | $-(CH_2)_7-$ | | |
| 270 | $4-C_2H_5O-C_6H_4-O-$ | $-(CH_2)_7-$ | | |
| 271 | $4-CF_3-C_6H_4-O-$ | $-(CH_2)_7-$ | | |
| 272 | $4-C_6H_5-C_6H_4-O-$ | $-(CH_2)_7-$ | | |
| 273 | $2,4,6-(Cl_3)_3-C_6H_2-O-$ | $-(CH_2)_7-$ | | |
| 274 | $2,4,6-(CH_3)_3-C_6H_2-O-$ | $-(CH_2)_7-$ | | |
| 275 | 1-Naphthyl-O- | $-(CH_2)_7-$ | | |
| 276 | 2-Naphthyl-O- | $-(CH_2)_7-$ | | |
| 277 | $4-(C_6H_5-CH_2-O)C_6H_4-O-$ | $-(CH_2)_7-$ | | |
| 278 | $C_6H_5-CH_2-O-$ | $-(CH_2)_7-$ | | |

Tabelle 1 (Fortsetzung)

| Verb.-Nr. | R | Y | Fp(°C) | IR($cm^{-1}$) |
|---|---|---|---|---|
| 279 | $C_6H_5$-O- | $-(CH_2)_8-$ | | |
| 280 | 2-Cl-$C_6H_4$-O- | $-(CH_2)_8-$ | | |
| 281 | 3-Cl-$C_6H_4$-O- | $-(CH_2)_8-$ | | |
| 282 | 4-Cl-$C_6H_4$-O- | $-(CH_2)_8-$ | | |
| 283 | 2-F-$C_6H_4$-O- | $-(CH_2)_8-$ | | |
| 284 | 3-F-$C_6H_4$-O- | $-(CH_2)_8-$ | | |
| 285 | 4-F-$C_6H_4$-O- | $-(CH_2)_8-$ | | |
| 286 | 2-$CH_3$-$C_6H_4$-O- | $-(CH_2)_8-$ | | |
| 287 | 3-$CH_3$-$C_6H_4$-O- | $-(CH_2)_8-$ | | |
| 288 | 4-$CH_3$-$C_6H_4$-O- | $-(CH_2)_8-$ | | |
| 289 | 2-$CH_3O$-$C_6H_4$-O- | $-(CH_2)_8-$ | | |
| 290 | 3-$CH_3O$-$C_6H_4$-O- | $-(CH_2)_8-$ | | |
| 291 | 4-$CH_3O$-$C_6H_4$-O- | $-(CH_2)_8-$ | | |
| 292 | 4-t-$C_4H_9$-$C_6H_4$-O- | $-(CH_2)_8-$ | | |
| 293 | 4-$C_2H_5O$-$C_6H_4$-O- | $-(CH_2)_8-$ | | |
| 294 | 4-$CF_3$-$C_6H_4$-O- | $-(CH_2)_8-$ | | |
| 295 | 4-$C_6H_5$-$C_6H_4$-O- | $-(CH_2)_8-$ | | |
| 296 | 4-($C_6H_5$-$CH_2$-O)-$C_6H_4$-O- | $-(CH_2)_8-$ | | |
| 297 | 2,4,6-$Cl_3$-$C_6H_2$-O- | $-(CH_2)_8-$ | | |
| 298 | 2,4,6-$(CH_3)_3$-$C_6H_2$-O- | $-(CH_2)_8-$ | | |
| 299 | 1-Naphthyl-O- | $-(CH_2)_8-$ | | |
| 300 | 2-Naphthyl-O- | $-(CH_2)_8-$ | | |
| 301 | $C_6H_5$-$CH_2$-O- | $-(CH_2)_8-$ | | |
| 302 | $C_6H_5$-O- | $-(CH_2)_9-$ | | |

Tabelle 1 (Fortsetzung)

| Verb.-Nr. | R | Y | Fp($^oC$) | IR($cm^{-1}$) |
|---|---|---|---|---|
| 303 | $C_6H_5$-O- | $-(CH_2)_{10}-$ | | |
| 304 | 2-Cl-$C_6H_4$-O- | $-(CH_2)_{10}-$ | | |
| 305 | 3-Cl-$C_6H_4$-O- | $-(CH_2)_{10}-$ | | |
| 306 | 4-Cl-$C_6H_4$-O- | $-(CH_2)_{10}-$ | | |
| 307 | 2-F-$C_6H_4$-O- | $-(CH_2)_{10}-$ | | |
| 308 | 3-F-$C_6H_4$-O- | $-(CH_2)_{10}-$ | | |
| 309 | 4-F-$C_6H_4$-O- | $-(CH_2)_{10}-$ | | |
| 310 | 2-$CH_3$-$C_6H_4$-O- | $-(CH_2)_{10}-$ | | |
| 311 | 3-$CH_3$-$C_6H_4$-O- | $-(CH_2)_{10}-$ | | |
| 312 | 4-$CH_3$-$C_6H_4$-O- | $-(CH_2)_{10}-$ | | |
| 313 | 2-$CH_3O$-$C_6H_4$-O- | $-(CH_2)_{10}-$ | | |
| 314 | 3-$CH_3O$-$C_6H_4$-O- | $-(CH_2)_{10}-$ | | |
| 315 | 4-$CH_3O$-$C_6H_4$-O- | $-(CH_2)_{10}-$ | | |
| 316 | 4-t-$C_4H_9$-$C_6H_4$-O- | $-(CH_2)_{10}-$ | | |
| 317 | 4-$C_2H_5O$-$C_6H_4$-O- | $-(CH_2)_{10}-$ | | |
| 318 | 4-$CF_3$-$C_6H_4$-O- | $-(CH_2)_{10}-$ | | |
| 319 | 4-$C_6H_5$-$C_6H_4$-O- | $-(CH_2)_{10}-$ | | |
| 320 | 4-($C_6H_5$-$CH_2$-O)-$C_6H_4$-O- | $-(CH_2)_{10}-$ | | |
| 321 | 2,4,6-$Cl_3$-$C_6H_2$-O- | $-(CH_2)_{10}-$ | | |
| 322 | 2,4,6-$(CH_3)_3$-$C_6H_2$-O- | $-(CH_2)_{10}-$ | | |
| 323 | 1-Naphthyl-O- | $-(CH_2)_{10}-$ | | |
| 324 | 2-Naphthyl-O- | $-(CH_2)_{10}-$ | | |
| 325 | $C_6H_5$-$CH_2$-O- | $-(CH_2)_{10}-$ | | |

Tabelle 1 (Fortsetzung)

| Verb.-Nr. | R | Y | Fp(°C) | IR($cm^{-1}$) |
|---|---|---|---|---|
| 326 | $3,4-Cl_2-C_6H_3$ | $-CH_2-$ | Harz | 2945, 1742, 1473, 1353, 1228, 1061, 1035, 819 |
| 327 | $3-C_6H_5O-C_6H_4$ | $-CH_2-$ | | |
| 328 | $4-C_{12}-H_{25}-C_6H_4$ | $-CH_2-$ | | |
| 329 | $2-Cl,6-CN-C_6H_3$ | $-CH_2-$ | | |
| 330 | $3,4-(CH_3)_2-C_6H_3$ | $-CH_2-$ | | |
| 331 | $2-CH_3,4-t-C_4H_9-C_6H_3$ | $-CH_2-$ | | |
| 332 | $2-Cl,4-CH_3O-C_6H_3$ | $-CH_2-$ | | |
| 333 | $2,3,6-Cl_3-C_6H_2$ | $-CH_2-$ | | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 2 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 3 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der

Lösung in Wasser erhält man eine wäßrige Dispersion.

III. 20 Gew.-Teile der Verbindung Nr. 4 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 6 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 13 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 19 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 3 werden mit 10 Gew.-Teilen Natriumsalz eines Phenolsulfonsäureharnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 4 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäureharnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde 2-Benzyloxyphenyl-glyoxylsäuremethylester-O-methyloxim (A) - bekannt aus EP 253 213 - benutzt.

Anwendungsbeispiel 1

Wirksamkeit gegen Botrytis cinerea an Paprika

Paprikasämlinge der Sorte "Neusiedler Ideal Elite" wurden, nachdem sich 4-5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22-24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe 6 und 13 bei der Anwendung als 0,05 %ige (Gew.%) Spritzbrühe eine bessere fungizide Wirkung zeigen (90 %) als der bekannte Vergleichswirkstoff A (65 %).

Anwendungsbeispiel 2

Wirkung gegen Pyrenophora teres

Gerstenkeimlinge der Sorte "Igri" wurden im Zweiblattstadium mit wäßrigen Suspensionen, die 80 % Wirkstoff und 20 % Emulgiermittel in der Trockensubstanz enthielten, tropfnaß gespritzt. Nach 24 Stunden wurden die Pflanzen mit einer Sporensuspension des Pilzes Pyronophora teres inokuliert und für 48 Stunden in eine Klimakammer mit hoher Luftfeuchte bei 18°C gestellt. Anschließend wurden die Pflanzen im Gewächshaus bei 20-22°C und 70 % relativer Luftfeuchtigkeit für weitere 5 Tage kultiviert. Dann wurde das Ausmaß der Symptomentwicklung ermittelt.

Das Ergebnis zeigt, daß die Wirkstoffe 2, 3, 4, 6, 13 und 19 bei der Anwendung als 0,05 %ige Spritzbrühe eine bessere fungizide Wirkung zeigen (90 %) als der bekannte Vergleichswirkstoff A (60 %).

**Patentansprüche**

1. Ortho-substituierte 1-Naphthylether der Formel I

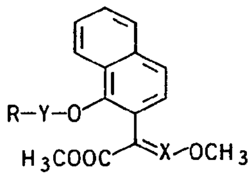

(I)

in der

X CH oder N bedeutet,

Y einen gegebenenfalls ungesättigten $C_1$-$C_{12}$-Alkylenrest und

R Aryl, Aryloxy, Arylthio oder Arylalkoxy bedeutet, wobei der aromatische Ring gegebenenfalls durch einen bis fünf der folgenden Reste substituiert ist: Halogen, $C_1$-$C_6$-Alkyl, $C_1$-$C_6$-Alkoxy, $C_1$-$C_4$-Alkylthio, $C_1$-$C_2$-Halogenalkyl, Aryl, Aryl-$C_1$-$C_2$-alkoxy, $C_1$-$C_4$-Alkylcarbonyl, durch $C_1$-$C_4$-Alkyl einfach oder doppelt substituiertes Amino, Cyano oder Nitro.

2. Verfahren zur Herstellung von neuen ortho-substituierten 1-Naphthylethern der Formel I gemäß Anspruch 1, dadurch gekennzeichnet, daß man ein Halogenid der allgemeinen Formel II,

R-Y-Hal (II)

in der R und Y die im Anspruch 1 genannten Bedeutungen haben und in der Hal für Chlor, Brom oder Jod steht, mit einer Verbindung der Formel IV,

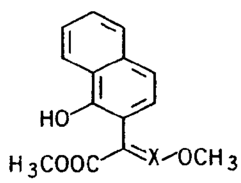

(IV)

in der X CH oder N bedeutet, umsetzt, oder eine Verbindung der Formel V

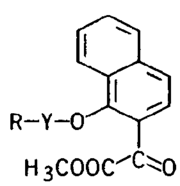

(V)

in der R und Y die in Anspruch 1 genannten Bedeutungen haben mit O-Methylhydroxylamin oder Hydroxylamin und einem Methylierungsmittel umsetzt.

3. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge einer Verbindung der Formel I behandelt,

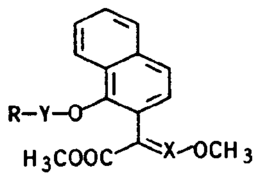

(I)

in der

X CH oder N bedeutet,

Y einen gegebenenfalls ungesättigten $C_1$-$C_{12}$-Alkylenrest und

R Aryl, Aryloxy, Arylthio oder Arylalkoxy bedeutet, wobei der aromatische Ring gegebenenfalls durch einen bis fünf der folgenden Reste substituiert ist: Halogen, $C_1$-$C_6$-Alkyl, $C_1$-$C_6$-Alkoxy, $C_1$-$C_4$-Alkylthio, $C_1$-$C_2$-Halogenalkyl, Aryl, Aryl-$C_1$-$C_2$-alkoxy, $C_1$-$C_4$-Alkylcarbonyl, durch $C_1$-$C_4$-Alkyl einfach oder doppelt substituiertes Amino, Cyano oder Nitro.

4. Fungizid, enthaltend einen inerten Trägerstoff und eine fungizid wirksame Menge einer Verbindung der Formel I

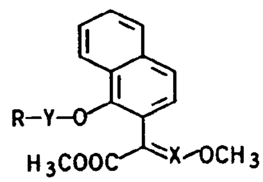

(I)

in der

X CH oder N bedeutet,

Y einen gegebenenfalls ungesättigten $C_1$-$C_{12}$-Alkylenrest und

R Aryl, Aryloxy, Arylthio oder Arylalkoxy bedeutet, wobei der aromatische Ring gegebenenfalls durch einen bis fünf der folgenden Reste substituiert ist: Halogen, $C_1$-$C_6$-Alkyl, $C_1$-$C_6$-Alkoxy, $C_1$-$C_4$-Alkylthio, $C_1$-$C_2$-Halogenalkyl, Aryl, Aryl-$C_1$-$C_2$-alkoxy, $C_1$-$C_4$-Alkylcarbonyl, durch $C_1$-$C_4$-Alkyl einfach oder doppelt substituiertes Amino, Cyano oder Nitro.

5. Verbindungen der Formel IV,

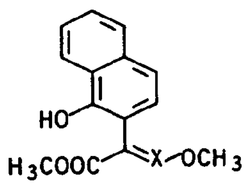

(IV)

in der X N bedeutet.

6. Verbindungen der Formel V,

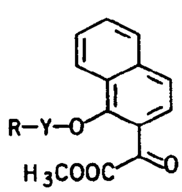

(V)

in der R und Y die in Anspruch 1 genannten Bedeutungen haben.

7. 1-Hydroxy-2-naphthylglyoxylsäuremethylester-O-methyloxim.

8. Verbindung gemäß Formel I in Anspruch 1, dadurch gekennzeichnet, daß R 2-Fluorphenyl Y $CH_2$ und X N bedeutet.

9. Verbindung gemäß Formel I in Anspruch 1, dadurch gekennzeichnet, daß R 2-Chlorphenyl Y $CH_2$ und X N bedeutet.

10. Verbindung gemäß Formel I in Anspruch 1, dadurch gekennzeichnet, daß R 2-Bromphenyl, Y $CH_2$ und X N bedeutet.

## Claims

1. An ortho-substituted 1-naphthyl ether of the formula I

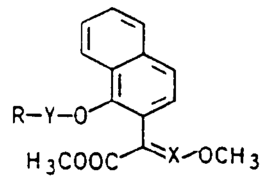

(I),

where

X is N,

Y is saturated or unsaturated $C_1$-$C_{12}$-alkylene and

R is aryl, aryloxy, arylthio or arylalkoxy, it being possible for the aromatic ring to be substituted one to five times by the following: halogen, $C_1$-$C_6$-alkyl, $C_1$-$C_6$-alkoxy, $C_1$-$C_4$-alkylthio, $C_1$-$C_2$-haloalkyl, aryl, aryl-$C_1$-$C_2$-alkoxy, $C_1$-$C_4$-alkylcarbonyl, amino which is substituted once or twice by $C_1$-$C_4$-alkyl, or cyano or nitro.

2. A process for preparing novel ortho-substituted 1-naphthyl ethers of the formula I as claimed in claim 1, which comprises reacting a halide of the formula II

R-Y-Hal (II)

where R and Y have the meanings specified in claim 1, and Hal is chlorine, bromine or iodine, with a compound of the formula IV

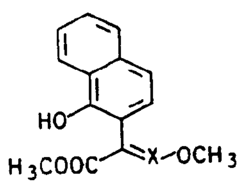

(IV)

where X is N, or reacting a compound of the formula V

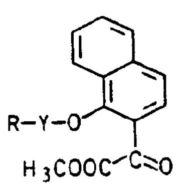

(V)

where R and Y have the meanings specified in claim 1, with O-methylhydroxylamine or hydroxylamine and a methyl-ating agent.

3. A method for controlling fungi, wherein the fungi or the materials, plants, seed or soil threatened by fungus attack are treated with a fungicidally effective amount of a compound of the formula I

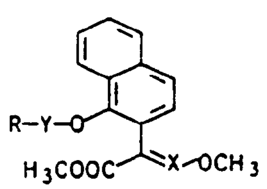

(I),

where

X is N,

Y is saturated or unsaturated $C_1$-$C_{12}$-alkylene and

R is aryl, aryloxy, arylthio or arylalkoxy, it being possible for the aromatic ring to be substituted one to five times by the following: halogen, $C_1$-$C_6$-alkyl, $C_1$-$C_6$-alkoxy, $C_1$-$C_4$-alkylthio, $C_1$-$C_2$-haloalkyl, aryl, aryl-$C_1$-$C_2$-alkoxy, $C_1$-$C_4$-alkylcarbonyl, amino which is substituted once or twice by $C_1$-$C_4$-alkyl, or cyano or nitro.

4. A fungicide containing an inert carrier and a fungicidally effective amount of a compound of the formula I

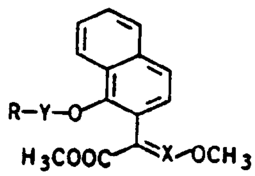

(I),

where

X is N,

Y is saturated or unsaturated $C_1$-$C_{12}$-alkylene and

R is aryl, aryloxy, arylthio or arylalkoxy, it being possible for the aromatic ring to be substituted one to five times by the following: halogen, $C_1$-$C_6$-alkyl, $C_1$-$C_6$-alkoxy, $C_1$-$C_4$-alkylthio, $C_1$-$C_2$-haloalkyl, aryl, aryl-$C_1$-$C_2$-alkoxy, $C_1$-$C_4$-alkylcarbonyl, amino which is substituted once or twice by $C_1$-$C_4$-alkyl, or cyano or nitro.

5. A compound of the formula IV

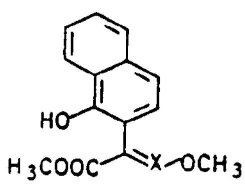

(IV)

where X is N.

6. A compound of the formula V

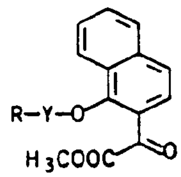

(V)

where R and Y have the meanings specified in claim 1.

7. Methyl 1-hydroxy-2-naphthylglyoxylate O-methyl-oxime.

8. A compound of the formula I as claimed in claim 1, where R is 2-fluorophenyl, Y is $CH_2$ and X is N.

9. A compound of the formula I as claimed in claim 1, where R is 2-chlorophenyl, Y is $CH_2$ and X is N.

10. A compound of the formula I as claimed in claim 1, where R is 2-bromophenyl, Y is $CH_2$ and X is N.

## Revendications

1. Ethers 1-naphtyliques substitués en ortho, de formule I

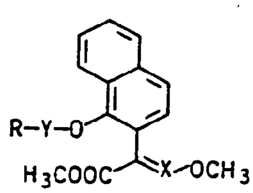

(I)

dans laquelle

X représente N,

Y représente un groupe alkylène en C 1-C 12 éventuellement insaturé, et

R représente un groupe aryle, aryloxy, arylthio ou arylalcoxy dans lesquels le noyau aromatique porte éventuellement de un à cinq des substituants suivants : des halogènes, des groupes alkyle en C 1-C 6, alcoxy en C 1-C 6, alkylthio en C 1-C 4, halogénoalkyle en C 1-C 2, aryle, aryl-alcoxy en C 1-C 2, (alkyle en C 1-C 4)-carbonyle, amino mono- ou di-substitué par des groupes alkyle en C 1-C 4, cyano ou nitro.

2. Procédé de préparation des nouveaux éthers 1-naphtyliques substitués en ortho de formule I de la revendication 1, caractérisé en ce que l'on fait réagir un halogénure de formule générale II

R-Y-Hal (II)

dans laquelle R et Y ont les significations indiquées dans la revendication 1 et Hal représente le chlore, le brome ou l'iode, avec un composé de formule IV

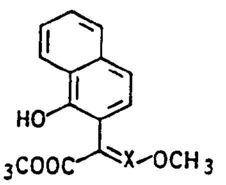

(IV)

dans laquelle X représente N, ou bien on fait réagir un composé de formule V

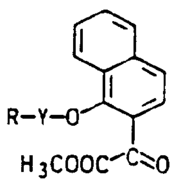

(V)

dans laquelle R et Y ont les significations indiquées dans la revendication 1, avec l'O-méthylhydroxylamine ou avec l'hydroxylamine et un agent méthylant.

3. Procédé pour combattre les mycètes, caractérisé en ce que l'on traite les mycètes ou les matières, végétaux, semences ou terrains menacés d'une attaque par les mycètes, par une quantité fongicide efficace d'un composé de formule I

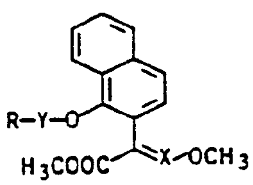

(I)

dans laquelle

X représente N,

Y représente un groupe alkylène en C 1-C 12 éventuellement insaturé, et

R représente un groupe aryle, aryloxy, arylthio ou arylalcoxy dans lesquels le noyau aromatique porte éventuellement un à cinq des substituants suivants : des halogènes, des groupes alkyle en C 1-C 6, alcoxy en C 1-C 6, alkylthio en C 1-C 4, halogénoalkyle en C 1-C 2, aryle, aryl-alcoxy en C 1-C 2, (alkyle en C 1-C 4)-carbonyle, amino mono- ou di-substitué par des groupes alkyle en C 1-C 4, cyano ou nitro.

4. Produit fongicide contenant un véhicule inerte et une quantité fongicide efficace d'un composé de formule I

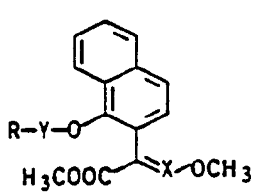

(I)

dans laquelle

X représente N,

Y représente un groupe alkylène en C 1-C 12 éventuellement insaturé, et

R représente en groupe aryle, aryloxy, arylthio ou arylacoxy dans lesquels le noyau aromatique porte éventuellement un à cinq des substituants suivants : des halogènes, des groupes alkyle en C 1-C 6, alkoxy en C 1-C 6, alkylthio en C 1-C 4,, halogénoalkyle en C 1-C 2, aryle, aryl-alcoxy en C 1-C 2, (alkyle en C 1-C 4)-carbonyle, amino mono- ou di-substitué par des groupes alkyle en C 1-C 4, cyano ou nitro.

5. Composés de formule IV

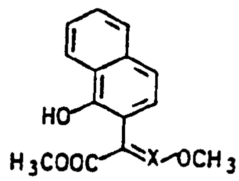

(IV)

dans laquelle X représente N.

6. Composés de formule V

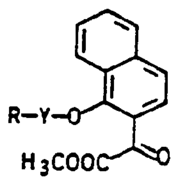

(V)

dans laquelle R et Y ont les significations indiquées dans la revendication 1.

7. L'O-méthyloxime du 1-hydroxy-2-naphtylglyoxylate de méthyle.

8. Composé de formule I de la revendication 1, caractérisé en ce que R représente un groupe 2-fluorophényle, Y représente $CH_2$ et X représente N.

9. Composé de formule I de la revendication 1, caractérisé en ce que R représente un groupe 2-chlorophényle, Y représente $CH_2$ et X représente N.

10. Composé de formule I de la revendication 1, caractérisé en ce que R représente un groupe 2-bromophényle, Y représente $CH_2$ et X représente N.